# EUROPÄISCHE PATENTSCHRIFT

(11) **EP 1 442 010 B1**
(45) Veröffentlichungstag und Bekanntmachung des Hinweises auf die Patenterteilung: **09.01.2008**
(21) Anmeldenummer: 02783019.9
(22) Anmeldetag: 28.10.2002
(51) Int. Cl.: C07C 253/04, C07C 255/50

(54) **VERFAHREN ZUR HERSTELLUNG VON BIPHENYL-4-CARBONITRIL**
METHOD FOR THE PRODUCTION OF BIPHENYL-4-CARBONITRILE
PROCEDE DE PRODUCTION DE BIPHENYL-4-CARBONITRILE

(30) Priorität: 30.10.2001 EP 01125852
(43) Veröffentlichungstag der Anmeldung: 04.08.2004
(73) Patentinhaber: Lonza AG, 4002 Basel (CH)
(72) Erfinder: BARTEK, Johannes, CH-3930 Visp (CH); WILLA, Pascal, CH-3945 Gampel (CH)
(74) Vertreter: Riegler, Norbert Hermann
(86) Internationale Anmeldenummer: PCT/EP2002/012008
(87) Internationale Veröffentlichungsnummer: WO 2003/037848

(56) Entgegenhaltungen:
- WO-A-96/16023
- DE-A- 2 737 210
- DE-A- 19 833 409

## Beschreibung

Die vorliegende Erfindung betrifft ein Verfahren zur Herstellung von Biphenyl-4-carbonitril der Formel

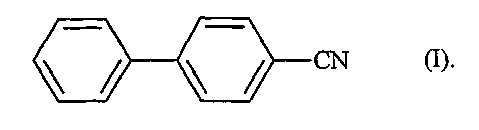

Biphenyl-4-carbonitril ist ein wichtiges Zwischenprodukt, beispielsweise in der Synthese von pharmazeutischen Wirkstoffen (siehe z. B. WO-A-01/22951).

DE-A-19833409 offenbart ein Verfahren zur Herstellung von Biphenyl-4-carbonitril durch Friedel-Crafts-Acylierung von 4-Hydroxybiphenyl.

Es sind zahlreiche Verfahren zur Herstellung von Biphenyl-4-carbonitril bekannt. Diese lassen sich im wesentlichen in drei Kategorien einordnen:
1. Austausch des Halogens in 4-Halobiphenylen gegen die Cyanogruppe
2. Umwandlung der funktionellen Gruppe in 4-substituierten Biphenylen in die Cyanogruppe
3. Kupplung von p-substituierten Benzonitrilen mit Phenylverbindungen

Diese Verfahren besitzen den Nachteil, dass sie teure Ausgangsmaterialien und/oder Reagenzien erfordern, die nicht in grossen Mengen verfügbar sind, und teilweise grosse Mengen schwermetallhaltiger Abfälle produzieren. Ausserdem liefern sie teilweise keine reinen Produkte, so ist beispielsweise ein in Kleinmengen kommerziell erhältliches Biphenyl-4-carbonitril (Aldrich) mit ca. 5% der Bromverbindung verunreinigt.

Aufgabe der vorliegenden Erfindung war es, ein Verfahren zur Herstellung von Biphenyl-4-carbonitril bereitzustellen, welches nur preiswerte und in grossen Mengen verfügbare Ausgangsmaterialien erfordert und keine problematischen Abfälle liefert. Erfindungsgemäss wird diese Aufgabe durch das Verfahren nach Patentanspruch 1 gelöst.

Es wurde überraschend gefunden, dass das in grossen Mengen verfügbare Biphenyl in Gegenwart von wasserfreiem Aluminiumchlorid mit Chlorcyan in guter Ausbeute und ohne nennenswerte Bildung von isomeren oder mehrfach cyanierten Nebenprodukten direkt zu dem gewünschten Biphenyl-4-carbonitril umgesetzt werden kann.

Das wasserfreie Aluminiumchlorid und das Chlorcyan werden vorzugsweise in einer Menge von jeweils 1,0 bis 1,5 mol auf 1 mol Biphenyl eingesetzt.

Das erfindungsgemässe Verfahren wird zweckmässig in einem inerten Lösungsmittel durchgeführt. Hierunter ist jedes Lösungsmittel zu verstehen, das unter den Reaktionsbedingungen nicht oder wesentlich langsamer als Biphenyl reagiert.
Vorzugsweise werden halogenierte aromatische Lösungsmittel wie beispielsweise Chlorbenzol, Brombenzol oder Dichlorbenzole eingesetzt. Besonders bevorzugt ist Chlorbenzol.

Das erfindungsgemässe Verfahren wird vorteilhaft bei Reaktionstemperaturen von ca. 60 bis 130 °C durchgeführt.

Die Reaktion kann auf verschiedene Weisen durchgeführt werden, beispielsweise kann das Biphenyl vorgelegt und dann zuerst das Aluminiumchlorid und dann das Chlorcyan zugegeben werden. Es ist aber auch möglich, das Aluminiumchlorid vorzulegen und dann zuerst das Chlorcyan einzuleiten und zuletzt das Biphenyl zuzugeben.

Die Aufarbeitung kann in der für Friedel-Crafts-Reaktionen mit Aluminiumchlorid üblichen Weise erfolgen, beispielsweise indem die Aluminiumsalze in viel Säure aufgelöst und so in die wässrige Phase gebracht oder mit wenig Wasser ausgefällt und abfiltriert werden.

Die folgenden Beispiele verdeutlichen die Durchführung des erfindungsgemässen Verfahrens, ohne dass darin eine Einschränkung zu sehen ist.

### Beispiel 1

In 200 ml Chlorbenzol wurden bei 25 °C unter Stickstoff 50 g (0,324 mol) Biphenyl gelöst. Anschliessend wurden 51,88 g (0,389 mol) wasserfreies Aluminiumchlorid zugegeben und für 15 min gerührt. Bei 20-25 °C wurden darauf 23,92 g (0,389 mol) Chlorcyan innerhalb einer Stunde eingeleitet. Nach beendeter Zugabe wurde noch 30 min bei 25 °C gerührt und anschliessend auf 110 °C erhitzt. Nach 4 h bei dieser Temperatur wurde die Reaktionslösung mit 200 ml Chlorbenzol verdünnt und auf 70-80 °C abgekühlt. Dann wurde die Reaktionslösung auf 600 ml eisgekühlte konzentrierte Salzsäure gegossen. Das Gemisch wurde filtriert und anschliessend die Phasen getrennt. Die wässrige Phase wurde noch einmal mit 100 ml Chlorbenzol extrahiert. Die vereinigten organischen Phasen wurden einmal mit 300 ml gesättigter Natriumhydrogencarbonatlösung gewaschen, über Natriumsulfat getrocknet und filtriert. Das Lösungsmittel wurde unter Vakuum abdestilliert und der so erhaltene Feststoff bei 50 °C/10 mbar für 24 h getrocknet. Man erhielt 54,0 g (93%) eines gelben Feststoffes, welcher nach Behandlung mit 3,0 g Aktivkohle aus Isopropanol/Wasser umkristallisiert wurde. Ausbeute: 41,8 g (72%) leicht gelbliche Kristalle.
¹³C NMR (CDCl₃): δ = 145,69 (s); 139,20 (s); 132,59 (s); 129,12 (s); 128,67 (s); 127,74 (s); 127,23 (s); 118,91 (s); 110,98 (s).

### Beispiel 2

In 200 ml Chlorbenzol wurden bei 25 °C unter Stickstoff 50 g (0,324 mol) Biphenyl gelöst. Anschliessend wurden 51,88 g (0,389 mol) wasserfreies Aluminiumchlorid zugegeben und für 15 min gerührt. Bei 20-25 °C wurden darauf 23,92 g (0,389 mol) Chlorcyan innerhalb einer Stunde eingeleitet. Nach beendeter Zugabe wurde das Reaktionsgemisch noch 30 min bei 25°C gerührt und anschliessend auf 110 °C erhitzt. Nach 4 h bei dieser Temperatur wurde die Reaktionslösung mit 200 ml Chlorbenzol verdünnt und auf 70-80 °C abgekühlt. Dann wurden innerhalb von 30 min 40 ml Wasser zugetropft. Anschliessend wurde das Gemisch auf 20°C abgekühlt und die ausgefallenen Aluminiumsalze abfiltriert. Der Filterrückstand wurde noch zweimal mit je 100 ml Chlorbenzol gewaschen. Die vereinigten organischen Phasen wurden eingedampft und der so erhaltene Rückstand wurde bei 50 °C/10 mbar getrocknet. Man erhält 52,8 g (91 %) eines gelben Feststoffes, welcher laut GC 90,5% Biphenyl-4-carbonitril enthielt.

### Beispiel 3

In 150 ml Chlorbenzol wurden bei 25 °C unter Stickstoff 51,88 g (0,389 mol) Aluminiumchlorid suspendiert. Anschliessend wurden innerhalb von 45 min 23,92 g (0,389 mol) Chlorcyan eingeleitet. Nach einer Nachreaktionszeit von 15 min wurde auf 90 °C erwärmt. Zur Suspension wurde dann innerhalb einer Stunde eine Lösung von 50 g (0,324 mol) Biphenyl in 50 ml Chlorbenzol zugetropft. Anschliessend wurde auf 110 °C gebracht und für 4 h reagieren gelassen. Dann wurde die Reaktionslösung innerhalb von einer Stunde auf 200 ml Wasser von 90 °C abgelassen. Man spülte das Reaktionsgefäss mit 100 ml Chlorbenzol nach und lies die Phasen separieren. Nach dem Ablassen der wässrigen Phase wurde die organische Phase bei 90 °C mit 200 ml Wasser gewaschen. Die so erhaltene organische Phase wurde anschliessend bei 40 °C mit 5 g Aktivkohle 30 min gerührt und dann filtriert. Anschliessend wurde die organische Phase zur Trockne eingedampft. Nach dem Trocknen bei 50 °C/10 mbar erhielt man 57,2 g (98,5%) eines gelben Feststoffes, welcher laut quantitativer ¹³C NMR-Analyse ca. 80% Biphenyl-4-carbonitril enthielt.

## Patentansprüche

1. Verfahren zur.Herstellung von Biphenyl-4-carbonitril der Formel 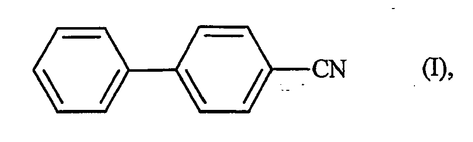 **dadurch gekennzeichnet, dass** Biphenyl in Gegenwart von wasserfreiem Aluminiumchlorid mit Chlorcyan umgesetzt wird.

2. Verfahren nach Anspruch 1, **dadurch gekennzeichnet, dass** das wasserfreie Aluminiumchlorid und das Chlorcyan jeweils in einer Menge von 1,0 bis 1,5 mol pro 1 mol Biphenyl eingesetzt werden.

3. Verfahren nach Anspruch 1 oder 2, **dadurch gekennzeichnet, dass** die Umsetzung in einem halogenierten aromatischen Lösungsmittel durchgeführt wird.

4. Verfahren nach Anspruch 3, **dadurch gekennzeichnet, dass** als halogeniertes aromatisches Lösungsmittel Chlorbenzol eingesetzt wird.

## Claims

1. A process for preparing biphenyl-4-carbonitrile of the formula 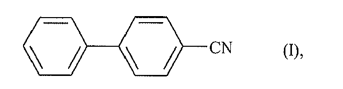 **characterized in that** biphenyl is reacted with cyanogen chloride in the presence of anhydrous aluminum chloride.

2. The process of claim 1, **characterized in that** the anhydrous aluminum chloride and the cyanogen chloride are each used in an amount of from 1.0 to 1.5 mol per 1 mol of biphenyl.

3. The process of claim 1 or 2, **characterized in that** the reaction is carried out in a halogenated aromatic solvent.

4. The process of claim 3, **characterized in that** the halogenated aromatic solvent used is chlorobenzene.

## Revendications

1. Procédé de production de biphenyl-4-carbonitrile de formule 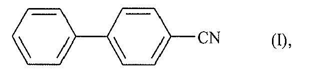 **caractérisé en ce que** le biphényle est mis à réagir avec du chlorure de cyanogène en présence de chlorure d'aluminium anhydre.

2. Procédé selon la revendication 1, **caractérisé en ce que** le chlorure d'aluminium anhydre et le chlorure de cyanogène sont utilisés dans les quantités, à chaque fois, de 1,0 à 1,5 mol par 1 mol de biphényle.

3. Procédé selon la revendication 1 ou 2, **caractérisé en ce que** la réaction est faite dans un solvant aromatique halogéné.

4. Procédé selon la revendication 3, **caractérisé en ce que** le solvant aromatique halogéné utilisé est le chlorobenzène.
